(19) **Europäisches Patentamt**

**European Patent Office**

**Office européen des brevets**

(11) **EP 0 684 227 B1**

(12) **EUROPEAN PATENT SPECIFICATION**

(45) Date of publication and mention
of the grant of the patent:
**24.06.1998 Bulletin 1998/26**

(51) Int. Cl.$^6$: **C07C 277/00**

(21) Application number: **95105591.2**

(22) Date of filing: **13.04.1995**

(54) **Process for the manufacture of guanidines**

Verfahren zur Herstellung von Guaniden

Procédé de préparation de guanidine

(84) Designated Contracting States:
**AT BE CH DE DK ES FR GB IT LI NL**

(30) Priority: **27.04.1994 EP 94106569**

(43) Date of publication of application:
**29.11.1995 Bulletin 1995/48**

(73) Proprietor:
**F. Hoffmann-La Roche AG**
**4002 Basel (CH)**

(72) Inventor: **Soukup, Milan**
**CH-4103 Bottmingen (CH)**

(74) Representative: **Mahé, Jean et al**
**F.Hoffmann-La Roche AG**
**Patent Department (PLP),**
**124 Grenzacherstrasse**
**4070 Basel (CH)**

(56) References cited:
**FR-A- 2 569 692**

• **JOURNAL OF THE AMERICAN CHEMICAL**
**SOCIETY., vol.75, no.16, 1953, GASTON, PA US**
**pages 4053 - 4054 F.L. SCOTT ET AL**
• **J.Org.Chem. 1992, 57,2497-2502**

## Description

A known process for the manufacture of guanidines, i.e. compounds containing the guanidino group -NC(NH)NH$_2$, consists in the amidination of a primary or secondary amine with 1-amidinopyrazole or amino imino methanesulfonic acid. Drawbacks of these amidination agents are their high price and the low yields resulting from their use. It has now been found that the amidination of primary or secondary amines can be performed in higher yields by utilizing the cheaper 1-amidino-1,2,4-triazol as a salt with a strong inorganic acid, e.g. with hydrochloric acid, as amidination agent.

The process of the invention can be performed with primary or secondary amines, more generally with compounds containing one or more primary or secondary amino groups NH$_2$ or NH and optionally other functional group, e.g. COOH. Where only one of the amino groups contained in a starting polyamine, e.g. a diamine, has to be converted into a guanidino group, the other amino group(s) must be protected. Such a selective protection of a primary amino group in the presence of a secondary one (which has to be selectively amidinated) can be performed by reacting the starting diamine with methyl acetoacetate in methanol in the presence of tributylamine.

Examples of primary amines on which the amidination process of the invention can be carried out are alkylamines, arylamines and aminoacids, e.g. tert-butylamine, aniline and glycine or alanine, respectively. Secondary starting amines are cyclic or acyclic, e.g. morpholine or piperidine, and diethylamine, respectively.

The amidination agent, e.g. 1-amidino-1,2,4-triazol-hydrochloride, can be prepared by reacting 1,2,4-triazol with cyanamid in the presence of hydrochloric acid in an apolar solvent, e.g. dioxane.

The amidination is readily performed in the presence of a base, such as tributylamine, diisopropylethylamine or sodium carbonate, in an aprotic or protic solvent, preferably in a lower alkanol, e.g. methanol or ethanol, or in DMF or DMSO, at a temperature up to reflux temperature of the reaction mixture, preferably at room temperature.

## Example 1

### A) The starting amine.

A)1. In a 0.5 l autoclave were added 100 g of 3-picolylamine, 100 ml of water and 5 g of 5%-rhodium on alumina. After degassing and establishing an internal hydrogen pressure of 40 bar, the reaction mixture was heated and stirred for 4 hrs at 50°C. After cooling to rt and flushing with inert atmosphere, the liquid was transferred in a 2 l flask and the autoclave was rinsed with water. The obtained aqueous solution of 3-aminomethyl-piperidine was evaporated under reduced pressure to a weight of 525 g, treated in one portion with 198.8 g (L)-O,O-dibenzoyltartaric acid and cooled to 28°C.

A)2. For crystallization of the resulting (L)-O,O-dibenzoyltartrate, 1 l of 2-propanol was added slowly under stirring. After stirring for 18 hrs, the crystals were filtered and washed with 2-propanol and finally dried under high vacuum at 45°C. The residue was dissolved under stirring at 75°C in 400 ml of water. This solution was cooled to 28°C and then treated with 1 l of 2-propanol under stirring over 3 hrs. After stirring overnight at rt, the crystals were filtered and washed with 2-propanol and finally dried under high vacuum at 45°C to yield 193.9 g (44.4%) of the (S)-3-aminomethyl-piperidine-(L)-O,O-dibenzoyltartrate, m.p. 146-148°C (dec.), $[\alpha]_D^{25}$ = -86.2° (c=1, water).

A)3. In a 2.5 l flask were added 100 g of L-O,O-dibenzoyltartrate product of paragraph A)2., 500 ml of water and 1 l of ethylacetate. The reaction mixture was stirred at rt and treated with 80 ml of 25% HCl. After stirring for 15 min, the organic phase was separated and extracted with water. The combined water phases were back extracted with ethylacetate and then evaporated under reduced pressure. The residue was dissolved in a mixture of ethanol and toluene and evaporated again under reduced pressure. To remove the last traces of water this procedure of dissolution and evaporation was repeated to yield 36.6 g (92.4%) of (S)-3-aminomethyl-piperidine-dihydrochloride, $[\alpha]_D^{25}$ = -3.6° (c=1, water).

### B) The amidination agent.

In a 1.5 l flask were added 41.4 g of 1,2,4-triazol, 25.3 g of cyanamid and 600 ml of dioxane. The resulting solution was stirred and heated to reflux. After the addition of 150 ml of 4.15N HCl in dioxane over 1 hr, the reaction mixture was further stirred under reflux for 1.5 hrs. The suspension was cooled slowly to rt and then stirred for 1 hr. The product was filtered, washed with dioxane and dried at 40°C under high vacuum to yield 85.9 g (97%) of 1-amidino-1,2,4-triazol-hydrochloride, m.p. 198-199°C.

### C) The process.

In a 1.5 l flask were added 36.6 g of the dihydrochloride of paragraph A)3., 300 ml of methanol, 93.3 ml of tributylamine and 23.2 ml of methyl acetoacetate. The suspension was stirred at rt for 5 hrs. As the reaction proceeded, the dihydrochloride dissolved slowly to give a clear solution. After addition of 46.7 ml tributylamine and of 28.9 g of 1-amidino-1,2,4-triazol-hydrochloride, the reaction solution was stirred at rt for 18 hrs and then treated at rt over 15 min with 65.2 ml of conc. HCl and subsequently stirred for further 45 min. The reaction mixture was evaporated under reduced pressure. To the residue were added 300 ml of ethanol and the suspension was stirred under reflux for 30 min and at rt overnight. The suspension was cooled to 0°C and stirred for 2 hrs before filtering the crystals which were washed with ethanol and dried in vacuum at rt. The crude material was suspended in ethanol and the mixture was refluxed and stirred for 30 min, then cooled to rt and stirred for 1 hr. The crystals were filtered, washed with ethanol, dried in vacuum at rt to yield 31.94 g colourless crystals (70%) of (S)-1-amidino-3-aminomethyl-piperidine-dihydochloride, m.p. 245-247°C, $[\alpha]_D^{25}$ = -17.4° (c=1, water).

Example 2

In a 100 ml flask were added 5 g of glycine, 9,83 mg of 1-amidino-1,2,4-triazol-hydrochloride and 7.06 g of sodium carbonate, dissolved in 166 ml of water. The reaction suspension was stirred at rt over night. The precipitate was filtered, washed with methanol and dried at rt under vacuum to give 8.08 g of amidinoglycine (79% yield), m.p. 279-283°C (dec.).

Example 3

In a 100 ml flask were added 5 g of piperidine, 7,59 g of diisopropylethylamine, 8.68 g of 1-amidino-1,2,4-triazol-hydrochloride and 30 ml of DMF. The slurry was stirred at rt over night. After addition of 10 ml of ether to the reaction solution, the product began to crystallize. The precipitate was filtered, washed with ethanol and dried at rt under vacuum to give 8 g of amidinopiperidine (82% yield), m.p. 180-182°C (dec.).

Example 4

In a 100 ml flask were added 5 g of aniline, 7.92 g of 1-amidino-1,2,4-triazol-hydrochloride and 13 ml of DMF. The slurry was stirred at 90°C over night. The reaction solution was treated with 10 ml of ether and stirred at -10°C over night. The crystals were filtered, washed with ether and then dried at rt under vacuum to give 6.1 g of phenylguanidine-hydrochloride (66% yield), m.p. 220-222°C (dec.).

**Claims**

1. A process for the manufacture of a guanidine by amidination of a primary or secundary amine, which process comprises reacting the amine with a salt of 1-amidino-1,2,4-triazol with a strong inorganic acid.

**Patentansprüche**

1. Verfahren zur Herstellung eines Guanidins durch Amidinierung eines primären oder sekundären Amins, welches Verfahren darin besteht dass man das Amin mit einem Salz des 1-Amidino- 1,2,4-triazols mit einer starken inorganischen Säure umsetzt.

**Revendications**

1. Procédé de fabrication d'une guanidine par amidination d'une amine primaire ou secondaire, lequel procédé comprend la réaction de l'amine avec un sel de 1-amidino-1,2,4-triazole avec un acide fort inorganique.